# EUROPEAN PATENT SPECIFICATION

(11) **EP 1 789 548 B1**
(45) Date of publication and mention of the grant of the patent: **19.12.2012**
(21) Application number: 05783876.5
(22) Date of filing: 19.09.2005
(51) Int. Cl.: C07K 16/18, C12N 15/10, C12N 15/13, C07K 16/00

(54) **ANTIBODY LIBRARIES**
ANTIKÖRPERBIBLIOTHEKEN
BIBLIOTHEQUES D'ANTICORPS

(30) Priority: 17.09.2004 GB 0420771
(43) Date of publication of application: 30.05.2007
(73) Proprietor: Randox Laboratories Ltd., Crumlin, County Antrim BT29 4QY (GB)
(72) Inventor: FITZGERALD, Peter, Randox Laboratories, Crumlin, County Antrim BT29 4QY (GB); LAMONT, John, Randox Laboratories Ltd, Crumlin, County Antrim BT29 4QY (GB); DUNLOP, Marshall, Randox Laboratories Ltd, Crumlin, County Antrim BT29 4QY (GB); SCHON, Shirley, Randox Laboratories Ltd, Crumlin, County Antrim BT29 4QY (GB)
(74) Representative: Jappy, John William Graham
(86) International application number: PCT/GB2005/003601
(87) International publication number: WO 2006/030238

(56) References cited:
- WO-A-01/27160
- WO-A-94/16094
- WO-A-98/18825
- WO-A-02/086085
- EWERT S ET AL: "Biophysical Properties of Human Antibody Variable Domains" JOURNAL OF MOLECULAR BIOLOGY, LONDON, GB, vol. 325, no. 3, 17 January 2003 (2003-01-17), pages 531-553, XP004457555 ISSN: 0022-2836 cited in the application
- LEE C V ET AL: "High-affinity Human Antibodies from Phage-displayed Synthetic Fab Libraries with a Single Framework Scaffold" JOURNAL OF MOLECULAR BIOLOGY, LONDON, GB, vol. 340, no. 5, 23 July 2004 (2004-07-23), pages 1073-1093, XP004518119 ISSN: 0022-2836
- DE BONO B ET AL: "VH Gene Segments in the Mouse and Human Genomes" JOURNAL OF MOLECULAR BIOLOGY, LONDON, GB, vol. 342, no. 1, 3 September 2004 (2004-09-03), pages 131-143, XP004844896 ISSN: 0022-2836
- WARD E SALLY: "VH shuffling can be used to convert an Fv fragment of anti-hen egg lysozyme specificity to one that recognizes a T cell receptor V-alpha" MOLECULAR IMMUNOLOGY, vol. 32, no. 2, 1995, pages 147-156, XP009057123 ISSN: 0161-5890
- KNAPPIK A ET AL: "Fully synthetic human combinatorial antibody libraries (HuCAL) based on modular consensus frameworks and CDRs randomized with trinucleotides" JOURNAL OF MOLECULAR BIOLOGY, LONDON, GB, vol. 296, no. 1, 11 February 2000 (2000-02-11), pages 57-86, XP004461525 ISSN: 0022-2836
- STEINHAUER C ET AL: "Single framework recombinant antibody fragments designed for protein chip applications" BIOTECHNIQUES 01 DEC 2002 UNITED STATES, vol. 33, no. 6 SUPPL., 1 December 2002 (2002-12-01), pages 38-45, XP001207669 ISSN: 0736-6205
- WU H ET AL: "Humanization of a murine monoclonal antibody by simultaneous optimization of framework and CDR residues" JOURNAL OF MOLECULAR BIOLOGY, LONDON, GB, vol. 294, no. 1, 19 November 1999 (1999-11-19), pages 151-162, XP004461859 ISSN: 0022-2836
- HANES J ET AL: "PICOMOLAR AFFINITY ANTIBODIES FROM A FULLY SYNTHETIC NAIVE LIBRARY SELECTED AND EVOLVED BY RIBOSOME DISPLAY" NATURE BIOTECHNOLOGY, NATURE PUBLISHING, US, vol. 18, no. 12, December 2000 (2000-12), pages 1287-1292, XP001121946 ISSN: 1087-0156
- CARTER P ET AL: "HUMANIZATION OF AN ANTI-P185HER2 ANTIBODY FOR HUMAN CANCER THERAPY" PROCEEDINGS OF THE NATIONAL ACADEMY OF SCIENCES OF USA, NATIONAL ACADEMY OF SCIENCE, WASHINGTON, DC, US, vol. 89, no. 10, 15 May 1992 (1992-05-15), pages 4285-4289, XP000275844 ISSN: 0027-8424
- REECK G R ET AL: "Homology in proteins and nucleic acids: a terminology muddle and a way out of it" CELL, CELL PRESS, CAMBRIDGE, NA, US, vol. 50, no. 5, 28 August 1987 (1987-08-28), page 667, XP002322075 ISSN: 0092-8674

### SEQUENCE LISTING

<110> Randox Laboratories Limited et al
<120> Antibody
<130> JWJ01119GB
<140> Not Yet Known
   <141> 2005-09-19
<150> GB0420771.8
   <151> 2004-09-17
<160> 2
<170> PatentIn version 3.1
<210> 1
   <211> 240
   <212> PRT
   <213> Artificial
<220>
   <223> Synthetic Antibody Fragment based on Murine Sequence
<220>
   <221> MISC_FEATURE
   <222> (99)..(99)
   <223> X represents the variable length VH CDR3, represented by dots in Figure 1.
<220>
   <221> MISC_FEATURE
   <222> (223)..(226)
   <223> VK CDR3 . X = Any amino acid
<400> 1
   Gln Val Gln Leu Gln Gln Ser Gly Ala Glu Leu Val Arg Ser Gly Ala
   1 5 10 15
   Ser Val Lys Leu Ser Cys Thr Ala Ser Gly Phe Asn Ile Lys Asp Tyr
   20 25 30
   Tyr Met His Trp Val Lys Gln Arg Pro Glu Gln Gly Leu Glu Trp Ile
   35 40 45
   Gly Trp Ile Asp Pro Glu Asn Gly Asp Thr Glu Tyr Ala Pro Lys Phe
   50 55 60
   Gln Gly Lys Ala Thr Met Thr Ala Asp Thr Ser Ser Asn Thr Ala Tyr
   65 70 75 80
   Leu Gln Leu Ser Ser Leu Ala Ser Glu Asp Thr Ala Val Tyr Tyr Cys
   85 90 95
   Asn Phe Xaa Asp Tyr Trp Gly Gln Gly Thr Thr Val Thr Val Ser Ser
   100 105 110
   Gly Gly Gly Gly Ser Gly Gly Gly Gly Ser Gly Gly Gly Gly Ser Asp
   115 120 125
   Val Leu Met Thr Gln Thr Pro Leu Thr Leu Ser Val Thr Ile Gly Gln
   130 135 140
   Pro Ala Ser Ile Ser Cys Lys Ser Ser Gln Ser Leu Leu Asp Ser Asp
   145 150 155 160
   Gly Lys Thr Tyr Leu Asn Trp Leu Leu Gln Arg Pro Gly Gln Ser Pro
   165 170 175
   Lys Arg Leu Ile Tyr Leu Val Ser Lys Leu Asp Ser Gly Val Pro Asp
   180 185 190
   Arg Phe Thr Gly Ser Gly Ser Gly Thr Asp Phe Thr Leu Lys Ile Asn
   195 200 205
   Arg Val Glu Ala Glu Asp Leu Gly Val Tyr Tyr Cys Trp Gln Xaa Xaa
   210 215 220
   Xaa Xaa Pro Leu Thr Phe Gly Ala Gly Thr Lys Leu Glu Ile Lys Glx
   225 230 235 240
<210> 2
   <211> 240
   <212> PRT
   <213> Artificial
<220>
   <223> Synthetic Antibody Fragment based on Murine Sequence
<220>
   <221> MISC_FEATURE
   <222> (99)..(99)
   <223> X represents the variable length VH CDR3, represented by dots in Figure 1.
<220>
   <221> MISC_FEATURE
   <222> (223)..(226)
   <223> VK CDR3. X = Any amino acid
<220>
   <221> MISC_FEATURE
   <222> (182)..(184)
   <223> VK CDR2. X = Any amino acid.
<400> 2
   Gln Val Gln Leu Gln Gln Ser Gly Ala Glu Leu Val Arg Ser Gly Ala
   1               5                   10                  15
   Ser Val Lys Leu Ser Cys Thr Ala Ser Gly Phe Asn Ile Lys Asp Tyr
               20                  25                  30
   Tyr Met His Trp Val Lys Gln Arg Pro Glu Gln Gly Leu Glu Trp Ile
           35                  40                  45
   Gly Trp Ile Asp Pro Glu Asn Gly Asp Thr Glu Tyr Ala Pro Lys Phe
       50                  55                  60
   Gln Gly Lys Ala Thr Met Thr Ala Asp Thr Ser Ser Asn Thr Ala Tyr
   65                  70                  75                  80
   Leu Gln Leu Ser Ser Leu Ala Ser Glu Asp Thr Ala Val Tyr Tyr Cys
                   85                  90                  95
   Asn Phe Xaa Asp Tyr Trp Gly Gln Gly Thr Thr Val Thr Val Ser Ser
               100                 105                 110
   Gly Gly Gly Gly Ser Gly Gly Gly Gly Ser Gly Gly Gly Gly Ser Asp
           115                 120                 125
   Val Leu Met Thr Gln Thr Pro Leu Thr Leu Ser Val Thr Ile Gly Gln
       130                 135                 140
   Pro Ala Ser Ile Ser Cys Lys Ser Ser Gln Ser Leu Leu Asp Ser Asp
   145                 150                 155                 160
   Gly Lys Thr Tyr Leu Asn Trp Leu Leu Gln Arg Pro Gly Gln Ser Pro
                   165                 170                 175
   Lys Arg Leu Ile Tyr Xaa Xaa Xaa Lys Leu Asp Ser Gly Val Pro Asp
               180                 185                 190
   Arg Phe Thr Gly Ser Gly Ser Gly Thr Asp Phe Thr Leu Lys Ile Asn
           195                 200                 205
   Arg Val Glu Ala Glu Asp Leu Gly Val Tyr Tyr Cys Trp Gln Xaa Xaa
           210                 215                 220
   Xaa Xaa Pro Leu Thr Phe Gly Ala Gly Thr Lys Leu Glu Ile Lys Glx
   225                 230                 235                 240

## Description

### Field of the invention

This invention relates to recombinant antibodies that are suitable for use in recombinant selection technologies and immobilisation onto a solid surface.

### Background to the invention

The field of proteomics relies on the ability to study large numbers of proteins, on a massive scale. Protein arrays, which contain a large number of proteins immobilised onto a solid support, are an invaluable tool in this field and allow high-throughput analysis of protein structure and function. A solid support suitable for the immobilisation of proteins in an array is described in EP-A-0874242.

Most applications of protein arrays require the array-bound proteins to bind specifically to a target molecule. Antibodies are the preferred ligands that are used in the majority of arrays.

In order for arrays to be functional, it is essential that the immobilised antibodies are stable and maintain their ability to bind target molecules. However, the process of immobilisation can inactivate an antibody, and only a small fraction of antibodies are suitable for immobilisation onto an array. The viability of the antibodies can also deteriorate during storage on the array surface. It is therefore recognised that engineering antibodies with characteristics suitable for immobilisation is desirable. Antibody fragments are the currently preferred targets for engineering, in particular Fab and single chain Fv (scFv) antibody fragments.

Single-chain Fv fragments, consisting of variable light chain (VL) and variable heavy chain (VH) regions connected by a linker, are antibody fragments that maintain specific binding ability. They can also be expressed as a single polypeptide chain. Their small size and relative ease of production in certain recombinant systems, compared with full-length antibodies, makes them a favourable choice for use in arrays. However, many scFv fragments are still unstable when immobilised, making them unsuitable for array-based technologies. Furthermore, scFvs often display poor soluble expression characteristics when expressed in E.coli, the preferred expression system for recombinant proteins. In particular, cytoplasmic E.coli expression is favoured as it allows a cost-effective production of high yields of recombinant proteins. However, the solubility of scFv fragments in this system is particularly limited, largely due to the reducing environment of the *E. coli* cytoplasm. A key requirement, therefore, for the cost effective production of scFv arrays is a collection of fragments that are stable upon immobilization to a solid support and demonstrate high yields of soluble expression in the cytoplasm of *E*. *coli.*

Protein array analysis is often carried out in conjunction with recombinant selection technologies such as phage and ribosome display, which allow for the selection of antibodies with specific binding affinity for a target molecule. These techniques are well known in the art, as discussed in Rodi et al, Current Opinions in Biotechnology, 1999; 10:87-93 and Hanes et al, FEBS letters, 1999; 450:105-110. Proteins that are identified by these techniques as binding a specific target molecule are ideal candidates for use as ligands on a protein array. It is therefore desirable that the proteins are stable when immobilized to a solid support, i.e. that the binding affinity of the protein is maintained after immobilization.

A number of groups have adopted the approach of choosing a protein framework (either an antibody scFv fragment or an alternative protein domain), with favourable stability and expression characteristics, and using that framework as the basis to build a library of fragments for use in recombinant selection technologies and subsequent array immobilisation. An example of a scFv framework that has suitable qualities required for antibodies used in arrays is human VH3-23/VL1-47 (Steinhauer et al, Biotechniques, 2002 Dec; Suppl:38-45). This fragment has demonstrated a long shelf life during storage on a number of microarray surfaces.

A recent study by Ewert et al, (J. Mol. Biol (2003) 325; 3: 531-553) was performed on the biophysical properties of human antibody variable domains, with the aim of identifying the physical properties of scFv frameworks and improving the design of scFv libraries. Due to their apparent advantages of solubility and stability, the authors recommended using a combination of a heavy chain from the human VH3 family in conjunction with a light chain from the human VK3 family (heavy and light chain families as characterised in the HuCAL database, Knappick et al, J. Mol. Bioi. (2000) 296: 57-86), when constructing an scFv framework.

The limited number of frameworks currently available are not sufficient to provide antibody fragments for all proteomic applications. For instance, it is envisaged that a variety of different scFv framework sequences will be necessary for use on different solid supports to provide varying biophysical characteristics or simply to increase the repertoire of structural variation within the library. There is therefore the need for further antibody frameworks that display good soluble expression and stability characteristics on different surfaces.

### Summary of the invention

The present invention is based on the surprising discovery that an antibody framework region based on a human VH1B heavy chain and a human VK2 light chain (heavy and light chain nomenclature as defined in the ImMunoGeneTics (IMGT) database) is solubly expressed in a microorganism and is stable, in particular when immobilised to a solid support. This framework is suitable for the generation of a library of antibody fragments with varied binding affinities. According to a first aspect of the invention, a composition comprises a plurality of antibody fragments, each comprising a framework region having a human VH1B heavy chain and a human VK2 light chain, or equivalent chains from other species, each antibody fragment further comprising at least one different CDR. It is surprising that these chains provide a stable framework, as Ewert *et al (supra)* teach that human VH3 and VK3, which are not equivalent to human VH1 B heavy chain and human VK2 light chain are the most suitable chains for creating soluble, stable scFv antibody fragments.

The library of antibody fragments according to the invention can be used in recombinant selection technologies to select for antibody fragments with affinity for a specific target molecule and which are suitable for immobilisation onto a solid surface.

According to a second aspect of the invention, a method for identifying an antibody having affinity for a target molecule comprises the steps of:
(i) treating a composition comprising a plurality of nucleic acids encoding a plurality of antibody fragments as defined in relation to the composition of the present invention; under conditions suitable for gene expression;
(ii) contacting the expressed antibody fragments with a target molecule;
(iii) identifying antibody fragments that bind to the target molecule; and
(iv) optionally, introducing variation into those nucleic acids encoding the identified antibody fragments and repeating steps (i) to (iii) to identify antibody fragments with different binding properties.

In a preferred embodiment, the steps (i) to (iii) are carried out by phage display or ribosome display or any combination thereof.

According to a third aspect of the invention, a method of creating a plurality of antibody fragments, each comprising a different binding affinity, comprises the step of mutating at least one CDR within a framework region having a human VH1B heavy chain and human VK2 light chain, or equivalent chains from other species, wherein each antibody fragment comprises a different mutation.

### Description of the Drawings

The invention is described with reference to the following drawings, wherein:

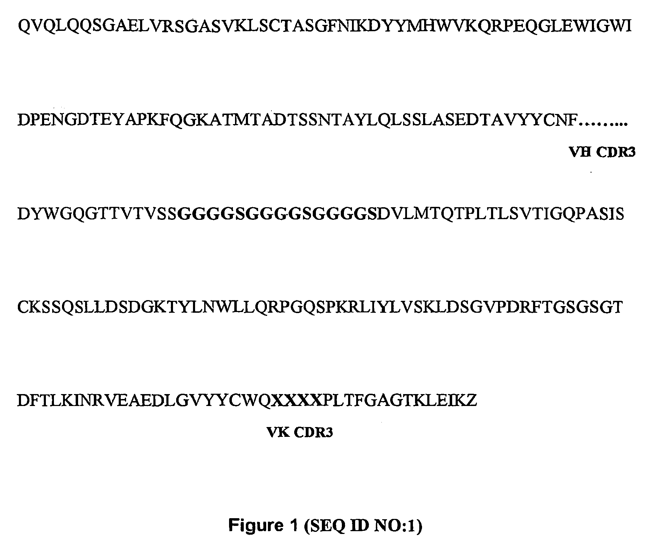

Figure 1 illustrates an scFv framework sequence with an N-terminal heavy chain belonging to the mvh14 heavy chain family followed by a (Giy4Ser)₃ linker followed by a C-terminal light chain of the mvk2 kappa chain family, the randomised residues of the VK CDR3 are represented by X and the variable length VH CDR3 is represented by dots; and

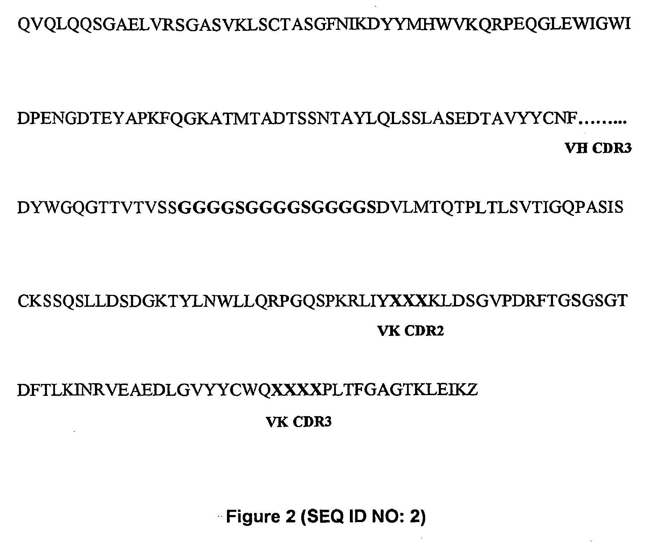

Figure 2 illustrates an identical scFv framework sequence as described in figure 1, but with the light chain CDR2 region additionally randomised, the randomised residues of the VK CDR2 and CDR3 regions are represented by X and the variable length VH CDR3 is represented by dots.

### Detailed Description of the Invention

The present invention identifies an antibody fragment that is expressed in soluble form and can retain stability when immbobilised on a solid support. The antibody fragment can be used as a framework to create an antibody library that contains antibodies with a number of different binding affinities, for different target molecules. Each antibody within the library maintains the favourable solubility and stability of the framework, but has at least one altered complementarity determining region (CDR), ensuring a range of different binding affinities.

As used herein, the term "antibody" refers to an immunoglobulin protein molecule that has the ability to bind a target molecule or antigen. Antibody fragments that maintain the ability to bind a target molecule, such as Fab and scFv fragments, are within the scope of the invention. The term "recombinant antibody" refers to an antibody molecule that has been created or altered by molecule cloning, as will be appreciated by one skilled in the art.

Antibody structure is well known and, although there are various structural classes of antibodies, for example, IgA, IgE, IgD, IgM and IgG in humans, they typically comprise heavy and light chains, each with variable and constant regions. Each heavy and light chain variable region contains three complementary determining regions (CDRs), which comprise a number of surface-exposed hyper-variable loops that are responsible for antigen-binding. Altering the structure of these hyper-variable lopps therefore alters the binding-specificity of the antibody fragment. Stable antigen-binding sites are formed by the combination of a variable region from a heavy chain and (VH) and a variable region from a light chain (VL). The VH and VL regions can be joined using a linker region to form a single recombinant polypeptide, known as a single chain Fv fragment, which retains antigen binding ability.

The present invention provides a framework antibody fragment that is solubly expressed and is stable, maintaining antigen binding ability, when immobilized to a solid support, for example as part of an array. The fragment contains a framework having human VH1B heavy chain and human VK2 light chain (as defined by the IMGT database), or equivalent chains from other species. The muring VH14 heavy chain and murine VK2 light chain sequences are illustrated as scFv fragments in Figures 1 and 2. As used herein, the term "framework" refers to the human VH1 B and VK2 chains, or equivalent chains from one species, excluding the amino acid residues in the CDRs that are identified herein as mutatable. The amino acid residues that are identified herein as mutatable in order to provide variation of CDR sequence and therefore binding affinity, may be mutated in any way. Provided that the non- mutatable residues that form the framework are not altered, the resulting fragment is likely to be stable and solubly expressed. The mutatable amino acid residues that are altered within the CDR will confer the antigen binding specificity.

Although the CDR regions are mutated, to ensure that antigen-binding ability is not lost, it is preferred that the residues within each CDR that stabilise the localised characteristic canonical structure, by bonding with other residues as described by Chothia et al J Mol Bioi. 1992 Oct 5;227(3):799-817, are not mutated, i.e. are conserved. As used herein, a residue that is "mutatable" is any residue located in a CDR that is not responsible for maintaining the structure of the CDR A "non-mutatable" residue is any residue within a CDR that is responsible for maintaining the CDR structure, or any non-CDR residue. Preferably, residues that are mutated are surface-exposed CDR residues that are not involved in the localised canonical structure. Residues situated at the apices of the hyper-variable loops are primarily favoured for mutation.

The invention results from the discovery that altering at least one CDR in the antibody framework allows the generation of antibody fragments that can be engineered to bind any target molecule, whilst maintaining the preferable stability and soluble expression characteristics of the framework. Although the work detailed herein has been performed with scFv fragments, one skilled in the art will appreciate that o ther types of recombinant antibody fragments, including but not limited to Fabs, diabodies or further scFv multimers, that contain the defined VH and VL regions, will also be stable and solubly expressed and are therefore within the scope of the invention.

Homologues of the human VH1B heavy chain and a human VK2 light chain, that provide similar stability and expression characteristics, are within the scope of the invention. Preferably, the homologues are equivalent chains from other species, including but not limited to the murine VH14 heavy chain and murine VK2 light chain (as defined in the HuCAL database). It is surprising that these chains provide a stable framework, as Ewert *et al (supra)* teach that human VH3 and VK3 (as defined by HuCAL) are most the most suitable chains for creating soluble, stable scFv antibody fragments.

The equivalent chains from other species have greater than 30% sequence identity, more preferably greater than 50% identity, even more preferably greater than 70% sequence identity and most preferably greater than 90% sequence identity to the human VH1 B and VK2 chains described herein, at the nucleic acid or amino acid level.

The concept of sequence identity is well known in the art, and refers to the level of identity between two amino acid or nucleic acid sequences. Equally well known is the concept of similarity, wherein the conservative differences between two sequences, which do not have a large effect on structure or function, are included when considering the likeness between two sequences. For example, a glutamic acid may be substituted for an aspartic acid without a large effect on the protein structure or function, these residues are "similar". In contrast, an aspartic acid residue shows no similarity to a phenylalanine residue. Equivalent chains from other species included within the scope of the invention must have as high similarity to the human VH1 B and VK2 chains. Identity and similarity may be calculated using any well known algorithm, form example Needleman-Wunsch, Smith-Waterman, BLAST or FASTA.

When calculating the identity or similarity of equivalent chains from other species, it is preferred that the amino acid residues within the CDRs, that are identified herein as mutable, are discounted and the homology only refers to the VH14 and VK2 framework region. The term "homology" is therefore intended to refer to similarity in the non-mutatable regions of the VH14 and VK 2 sequences only. This is because the residues within the CDRs identified herein as mutatable are intended to be altered and do not contribute significantly to the stability or solubility of the fragment. Including them in the homology calculation will therefore not reflect the region of the antibody which provides the desirable characteristics. It is also preferable to discount the linker segment in the scFv fragment when calculating homology, for the same reasons.

At least one residue in at least one CDR of the recombinant antibody is altered. As there are 3 CDR regions in each VH and VL region, one or more of the VH CDR1, VK CDR1, VH CDR2, VK CDR2, VH CDR3 and VK CDR3 regions may be mutated. The mutation of CDR regions, whilst leaving the non-CDR residues unaffected, is illustrated by Figures 1 and 2. Methods of altering residues within a recombinant molecule are well known in the art, for example site directed mutagenesis. Any mutation may be introduced, including an addition, substitution or deletion of at least one amino acid residue. Specific residues may be mutated in a targeted strategy, or mutatable residues in each CDR may be randomly mutated.

Preferably, at least one CDR region is randomised, excluding those residues identified as non-mutatable, to generate a potentially novel binding site. As used herein, the term "randomised" refers to a random selection of mutatable amino acid residues in the CDR being randomly mutated to any other amino acid residue.

It is preferred that at least one of the CDRs has at least one extra residue incorporated, to increase the variation of sequence generated. More preferably, between 2 and 10 extra residues are incorporated, most preferably 4 extra residues. The extra residues may be incorporated into any CDR, although incorporation into the VH CDR3 region mimics the extra variation naturally found in this region of an immunoglobulin molecule.

The antibody fragment is solubly expressed *in vivo* and is stable, maintaining antigen binding ability, when immobilised on a solid support. By "solubly expressed", it is meant that the antibodies do not aggregate excessively or form inclusion bodies in the host expression system. Expression of recombinant antibodies is usually carried out in an *E.coli* system, although any expression system is within the scope of the invention, including yeast and other eukaryotic systems. In a preferred embodiment, the antibody is solubly expressed in the cytoplasm of *E*. *coli.*

Antibody fragments of the invention are created by standard molecular cloning procedures. The recombinant polynucleotides that code for the antibodies of the invention are within the scope of the invention. As used herein the term "polynucleotide" refers to a series of linked nucleic acids, including but not limited to RNA, DNA, PNA (peptide nucleic acid) or LNA (locked nucleic acid). Nucleic acid mimics are also within the scope of the invention.

A library of antibodies can be created, containing a large number of different binding affinities. Each antibody fragment within the library contains at least one CDR that is different to other antibodies in the library. Each CDR is altered by mutating at least one amino acid residue, as described above, thereby generating a large number of different binding affinities. Depending on the desired use of the library, the library may contain a range of affinities directed towards a single molecule or class of molecules, or may contain a large, random, range of affinities. In a preferred embodiment, a library is created by randomisation of at least one CDR, as described above. To generate the maximum variation within the library, each CDR is randomised. As will be appreciated by one skilled in the art, this will provide a library containing an extensive number of binding affinities, to a diverse range of target molecules. Each antibody in the library, although having a substantially unique binding affinity, will still contain the favourable soluble expression and stable immobilisation characteristics due to the common framework shared by all members of the library, by merit of the careful choice of residues that are to be mutated.

An antibody of the invention may be used in an assay to detect binding to a target molecule. Such immunoassays are well known in the art, the most common of which is ELISA.

A library of antibody fragments, or a library of recombinant nucleic acids that codes for the library of antibodies, can be used in recombinant selection technologies in order to select for antibodies within the library that bind to a specific target molecule. Briefly, these selection techniques rely on the expression of a library of antibodies with a variety of different binding affinities. The expressed library is then contacted with at least one target molecule, and any antibody that binds the target molecule is identified and further investigated. Several rounds of selection may be required in order to identify an antibody with the desired binding characteristics. An antibody identified by a recombinant selection technique may be further mutated or altered in order to introduce further variation that may result in an antibody fragment with an optimal binding affinity. Any recombinant selection technology may be used, including ribosome display and phage display which are well known in the art.

The ribosome display technique results essentially in the enrichment of specific mRNAs by immunoprecipitation of ribosome complexes. This operates by translating a library of antibodies according to the present invention *in vitro* under conditions that maintain a complex between the translated polypeptide, the mRNA and the translating ribosome. Maintaining the mRNA, polypeptide and ribosome complex can be achieved by conventional methods, e.g. by the absence of a stop codon, or the inclusion of specific agents which block prokaryotic translation, e.g rifampicin or chloramphenicol. Additional buffer components can also be used to increase the stability of the complex. The DNA library is transcribed into RNA which is then translated *in vitro* to produce the first polypeptide library. Translation may be stopped by cooling on ice and the desired ribosome complexes can be selected by affinity with a target ligand immobilised on a solid support material. Non-specific ribosome complexes can be removed by intensive washing and the bound ribosome complexes can then be disassociated, e.g. by the use of EDTA. The RNA may then be isolated from the complexes, reverse transcribed to cDNA, and re-amplified by the polymerase reaction, initiating a further cycle of the ribosome display technique.

The ribosome display technique is typically carried out in an optimised *Escherichia coli* system, although other systems, including rabbit reticulocyte ribosome display systems are also used. The rabbit reticulocyte system employs a coupled transcription/translation system, i.e. without the need to purify mRNA prior to translation, and may therefore be beneficial in some experiments. A description of the ribosome display techniques is disclosed in Hanes et al., Proceedings National Academy of Sciences USA, 1997; 94:4937-4942, and He et al., Nucleic Acids Research, 1997; 25(24):5132-5134.

The components necessary to carry out the ribosome display will be apparent to the skilled person, based on conventional methodologies. In particular, the conditions necessary to carry out the polymerase reaction, including the selection of the appropriate polymerase enzyme, will be apparent to the skilled person. The primers required in the polymerase reaction can be designed according to the specific DNA intended to be amplified.

Phage display is carried out by inserting DNA into a structural gene of the virus selected as the biological vehicle. Any appropriate biological vehicle may be used, including λ phage, T4 phage and bacteriophage. In a preferred embodiment, the M13 bacteriophage is used. According to the present invention, the library of antibodies with a variety of binding affinities are transformed into the phages. If the insertion of the DNA does not disrupt the essential functions of the gene product, the mutation-encoded peptide will be displayed on the surface of a viral particle. This may then be identified and isolated from other displayed peptides, on the basis of binding affinity with a suitable target ligand. The insertion of the DNA recombinant library into the viral genome can be carried out by conventional methods known to those skilled in the art. The recombinant viruses may then be used to transfect a suitable host cell, e.g. *E*. *coli* to promote viral replication and translation of the incorporated DNA. The screening process may then take place, to identify those proteins of particular interest. The identity of the selected proteins can be found by determining the DNA sequence of the selected phage at the sites of new DNA insertion. The identified DNA sequences can then be used to design further phage-display libraries that will further improve the binding properties of the selected proteins.

Any antibody according to the present invention that is identified by a recombinant selection technique as binding to a target molecule will be solubly expressed and will retain its ability to bind specifically that target molecule when immobilised to a solid support material. A single antibody may be immobilised onto a solid support, or a number of antibodies, thereby forming an array, which may form a random or ordered pattern on the solid support. Preferably, the arrays that are used are single molecule arrays that comprise antibodies in distinct optically resolvable areas.

Methods of immobilising proteins to solid support surfaces are well known in the art. Immobilisation may be by specific covalent or non-covalent interactions. Suitable support materials are known in the art, and include glass slides, ceramic and silicon surfaces and plastics materials. The support is usually a flat (planar) surface. In a preferred embodiment, an antibody according to the present invention is covalently attached to a support as described in EP-A-0874242.
The invention is further illustrated by the following Example.

### Example

A large number of candidate scFv fragments were characterised for both solubility and stability. Of these, a mouse anti-p53 scFv fragment was selected as a suitable candidate as the basis for construction of an scFv framework library. The fragment was selected on the basis of its high levels of soluble expression in E.coli, as determined in a quantitative ELISA assay, and its functional stability on both micro-array chips and other surfaces.

The antibody framework was constructed in an scFv format with an N-terminal heavy chain belonging to the mvh14 heavy chain family followed by a (Gly4Ser)₄ linker followed by a C-terminal light chain of the mvk2 kappa chain family, as illustrated by figures 1 and 2. The heavy and light chain family nomenclature used is as described by the ImMunoGeneTics (IMGT) Database on the world wide web. The framework fragment was constructed by recursive PCR synthesis (Prodromou & Pearl, Protein Eng. 1992 Dec;S(8):827-9), based upon the sequence of an anti-p53 antibody from a hybridoma, identified by Jannot & Hynes Biochem Biophys Res Commun. 1997 Jan 13;230(2):242-6.

The sequence variation was initially introduced at pre-determined residues in CDR3 of both the heavy and light chains, by site-directed mutagenesis. The regions mutated are illustrated in bold in figure 1. In the heavy chain CDR3 , further variation was introduced by the incorporation of additional randomised residues, mimicking the extra variation naturally found in the VH CDR3 of the immunoglobulin molecule. In total up to four extra residues were incorporated, forming a sequence of between five and nine random residues at the heavy chain CDR3.

This initial library has been validated by screening of the library for soluble expression in *E*. *coli* BL21 (DE3) pLysS cells. A high percentage of the fragments were shown to be expressed in a soluble form. In addition, six clones were DNA sequenced, with the sequence data demonstrating that all of the sequenced clones had both full length reading frames and fully randomised amino acids at the correct residues. A further six clones were subsequently chosen at random and expressed alongside the anti-p53 scFv. These fragments were tested by ELISA and biochip assays. The anti-p53 scFv fragment performed as expected, demonstrating the usual binding signals. The expressed fragments from the anti-p53 derived framework library with randomised CDRs, however, did not show any binding signal in the presence of p53 antigen, suggesting that their specificity had been changed by the randomization process.

Subsequent to this work a second framework library was created, whereby, selected residues of the light chain CDR 2 were randomised in addition to the two CDR 3 regions which were randomised in the first library. The residues randomised in this second library are illustrated in bold in Figure 2. From this library, 60 clones were expressed in 10 ml expression cultures. These cultures were subsequently lysed and purified in a one-step IMAC purification system. Of the 60 clones, 48 expressed full length products of which 26 (54%) were classified as soluble, as defined using the criteria that a band of the correct size could be observed in the purified eluate when stained with Coomassie stain (Sambrook, Fritsch and Maniatis, Molecular Cloning, 1989, Cold Spring Harbour Laboratory Press). Typically these bands accounted for over 20% of the total purified eluate protein as quantified visually from the Coomassie staining. From the 60 Clones, a selection of 20 random clones were sequenced. The sequence data showed that the three designated CDRs had been mutated in all 20 clones sequenced. The 3 clones within this group which had not expressed a full length protein, had incorporated deletions into one of the CDRs which had been randomized during the PCR process.

## Claims

1. A composition comprising a plurality of antibody fragments, each comprising a framework region having a human VH1 B heavy chain and a human VK2 light chain, or equivalent chains from other species, each antibody fragment further comprising at least one different CDR.

2. A composition according to claim 1, wherein each framework region consists of a human VH1 B heavy chain and a human VK2 light chain, or equivalent chains from other species.

3. A composition according to claim 1 or claim 2, wherein the equivalent chains are murine.

4. A composition according to claim 3 wherein the framework comprises either of the sequences identified herein as SEQ ID Nos. 1 and 2.

5. A composition according to any preceding claim, wherein each antibody fragment is a single chain Fv fragment or multimer thereof.

6. A composition according to any preceding claim, wherein the different CDRs differ with respect to each other by the substitution, addition or deletion of at least one amino acid residue.

7. A composition according to claim 6, wherein each CDR retains those amino acid residues that stabilise the localised canonical structure of each CDR.

8. A composition comprising a plurality of nucleic acids encoding a plurality antibody fragments according to any of claims 1 to 7.

9. A method for identifying an antibody having affinity for a target molecule, comprising the steps of:
(i) treating the composition according to claim 8 under conditions suitable for gene expression;
(ii) contacting the expressed antibody fragments with a target molecule;
(iii) identifying antibody fragments that bind to the target molecule; and
(iv) optionally, introducing variation into those nucleic acids encoding the identified antibody fragments and repeating steps (i) to (iii) to identify antibody fragments with different binding properties.

10. A method according to claim 9, wherein steps (i) to (iii) are carried out by ribosome or phage display or any combination thereof.

11. A plurality of antibody fragments as defined in claim 1, immobilised on a support material.

12. A method of creating a plurality of antibody fragments, each comprising a different binding affinity, comprising the step of mutating at least one CDR within a framework region having a human VH1 B heavy chain and a human VK2 light chain, or equivalent chains from other species, wherein each antibody fragment comprises a different mutation.

## Patentansprüche

1. Zusammensetzung, umfassend eine Vielzahl von Antikörperfragmenten, wobei jedes eine Framework-Region mit einer humanen VH1B-Schwerkette und einer humanen VK2-Leichtkette oder gleichwertigen Ketten von anderen Spezies umfasst, wobei jedes Antikörperfragment ferner mindestens eine unterschiedliche CDR umfasst.

2. Zusammensetzung nach Anspruch 1, wobei jede Framework-Region aus einer humanen VH1B-Schwerkette und einer humanen VK2-Leichtkette oder gleichwertigen Ketten von anderen Spezies besteht.

3. Zusammensetzung nach Anspruch 1 oder 2, wobei die gleichwertigen Ketten murine Ketten sind.

4. Zusammensetzung nach Anspruch 3, wobei das Framework eine der beiden der hierin als SEQ ID NO: 1 und 2 identifizierten Sequenzen umfasst.

5. Zusammensetzung nach einem der vorangehenden Ansprüche, wobei jedes Antikörperfragment ein Einzelketten-Fv-Fragment oder Multimer davon ist.

6. Zusammensetzung nach einem der vorangehenden Ansprüche, wobei sich die unterschiedlichen CDR in Bezug aufeinander durch die Substitution, Addition oder Deletion von mindestens einem Aminosäurerest unterscheiden.

7. Zusammensetzung nach Anspruch 6, wobei jede CDR die Aminosäurereste beibehält, welche die lokalisierte kanonische Struktur von jeder CDR stabilisieren.

8. Zusammensetzung, umfassend eine Vielzahl von Nukleinsäuren, die für eine Vielzahl von Antikörperfragmenten nach einem der Ansprüche 1 bis 7 kodieren.

9. Verfahren zum Identifizieren eines Antikörpers mit einer Affinität für ein Targetmolekül, das die folgenden Schritte umfasst:
(i) Behandeln der Zusammensetzung nach Anspruch 8 unter Bedingungen, die zur Genexpression geeignet sind;
(ii) Inkontaktbringen der exprimierten Antikörperfragmente mit einem Targetmolekül;
(iii) Identifizieren von Antikörperfragmenten, die an das Targetmolekül binden; und
(iv) gegebenenfalls Einführen einer Variation in die Nukleinsäuren, die für die identifizierten Antikörperfragmente kodieren und Wiederholungsschritte (i) bis (iii) zum Identifizieren von Antikörperfragmenten mit unterschiedlichen Bindungseigenschaften.

10. Verfahren nach Anspruch 9, wobei die Schritte (i) bis (iii) durch Ribosomen- oder Phagen-Display oder jedwede Kombination davon durchgeführt werden.

11. Vielzahl von Antikörperfragmenten wie nach Anspruch 1 definiert, die auf einem Trägermaterial immobilisiert sind.

12. Verfahren zum Erzeugen einer Vielzahl von Antikörperfragmenten, wobei jedes aus einer unterschiedlichen Bindungsaffinität besteht, umfassend den Schritt des Mutierens von mindestens einer CDR in einer Framework-Region mit einer humanen VH1B-Schwerkette und einer humanen VK2-Leichtkette oder gleichwertigen Ketten von anderen Spezies, wobei jedes Antikörperfragment eine unterschiedliche Mutation umfasst.

## Revendications

1. Composition comprenant une pluralité de fragments d'anticorps, chacun contenant une région de charpente présentant une chaîne lourde humaine VH1B et une chaîne légère humaine VK2 ou des chaînes équivalentes provenant d'autres espèces, chaque fragment d'anticorps contenant en outre au moins une région de détermination de complémentarité (CDR) différente.

2. Composition selon la revendication 1, dans laquelle chaque région de charpente est constituée d'une chaîne lourde humaine VH1B et d'une chaîne légère humaine VK2 ou de chaînes équivalentes provenant d'autres espèces.

3. Composition selon la revendication 1 ou la revendication 2, dans laquelle les chaînes équivalentes sont murines.

4. Composition selon la revendication 3 dans laquelle la charpente renferme l'une des séquences identifiées dans l'invention comme constituée par SEQ ID Nos. 1 et 2.

5. Composition selon une quelconque revendication précédente, dans laquelle chaque fragment d'anticorps est un fragment Fv de chaîne unique ou un multimère de celui-ci.

6. Composition selon une quelconque revendication précédente, dans laquelle les différentes CDR diffèrent les unes par rapport aux autres par la substitution, l'addition ou la suppression d'au moins un résidu d'acide aminé.

7. Composition selon la revendication 6, dans laquelle chaque CDR conserve ceux des résidus d'acides aminés qui stabilisent la structure canonique localisée de chaque CDR.

8. Composition comprenant une pluralité d'acides nucléiques codant une pluralité de fragments d'anticorps selon l'une quelconque des revendications 1 à 7.

9. Procédé d'identification d'un anticorps ayant une affinité pour une molécule cible, comprenant les étapes consistant à :
(i) traiter la composition selon la revendication 8 sous des conditions appropriées à l'expression de gènes ;
(ii) mettre en contact les fragments d'anticorps exprimés avec une molécule cible ;
(iii) identifier des fragments d'anticorps qui se lient à la molécule cible ; et
(iv) facultativement, introduire une variation dans ceux des acides nucléiques codant les fragments d'anticorps identifiés et répéter les étapes (i) à (iii) afin d'identifier des fragments d'anticorps présentant différentes propriétés de liaison.

10. Procédé selon la revendication 9, dans lequel les étapes (i) à (iii) sont réalisées par exposition du ribosome ou du phage ou d'une combinaison de ceux-ci.

11. Pluralité de fragments d'anticorps tels que définis dans la revendication 1, immobilisés sur une matière support.

12. Procédé de création d'une pluralité de fragments d'anticorps, chacun comprenant une différente affinité de liaison, comprenant l'étape de mutation d'au moins une CDR au sein d'une région de charpente présentant une chaîne lourde humaine VH1B et une chaîne légère humaine VK2 ou des chaînes équivalentes provenant d'autres espèces, dans lequel chaque fragment d'anticorps comprend une mutation différente.
